# EUROPÄISCHE PATENTSCHRIFT

(11) **EP 3 218 065 B1**
(45) Veröffentlichungstag und Bekanntmachung des Hinweises auf die Patenterteilung: **04.12.2019**
(21) Anmeldenummer: 15778252.5
(22) Anmeldetag: 02.10.2015
(51) Int. Cl.: A61Q 5/08, A61Q 5/10, A61K 8/02, B65D 65/46, A61K 8/73, A61K 8/81

(54) **MITTEL UND VERFAHREN ZUR FÄRBUNG KERATINHALTIGER FASERN**
COMPOSITIONS AND METHODS FOR THE COLOURING KERATINIC FIBRES
AGENT ET PROCÉDÉ POUR COLORER DES FIBRES KERATINIQUES

(30) Priorität: 12.11.2014 DE 102014223093
(43) Veröffentlichungstag der Anmeldung: 20.09.2017
(73) Patentinhaber: Henkel AG & Co. KGaA, 40589 Düsseldorf (DE)
(72) Erfinder: ERKENS, Udo, 47877 Willich (DE); MÜLLER, Burkhard, 40221 Düsseldorf (DE)
(86) Internationale Anmeldenummer: PCT/EP2015/072830
(87) Internationale Veröffentlichungsnummer: WO 2016/074853

(56) Entgegenhaltungen:
- WO-A1-91/01127
- WO-A2-02/060980
- US-A- 3 892 905
- US-A- 4 156 047
- US-A1- 2006 002 965

## Beschreibung

Die Erfindung betrifft ein kosmetisches Mittel umfassend eine in einer wasserlöslichen und/oder wasserdispergierbaren Umhüllung verpackte kosmetische Zubereitung und ein Verfahren zum Färben keratinhaltiger Fasern.

Im Bereich der dekorativen Kosmetik, insbesondere der bleichenden und färbenden Haarkosmetik, besteht ein großer Bedarf an effektiven und gleichzeitig einfach und gefahrlos handhabbaren Produkten. Insbesondere auf dem Gebiet der Haarkosmetik stehen dem Verbraucher Blondier- und Haarfärbesysteme zur Verfügung, die äußerst effektiv wirken, jedoch bei unsachgemäßer Handhabung, beispielsweise bei Kontakt mit Hautpartien oder Augen zu Irritationen oder in Extremfällen sogar zum Auslösen von Allergien führen können. Es besteht daher ein großer Bedarf, die sichere Handhabbarkeit solcher kosmetischer Formulierungen zu gewährleisten und darüber hinaus dem Verbraucher ein einfach zu dosierendes Verpackungssystem an die Hand zu geben, das auch ein Anmischen bzw. eine Zusammenstellung der benötigten Komponenten vor Ort ermöglicht. Ein wesentlicher Punkt, insbesondere in Bezug auf bleichende oder färbende Haarkosmetika ist dabei der Vermeidung von Produktstäuben.

In der Literatur finden sich erste Ansätze zur Lösung der zuvor beschriebenen technischen Probleme. So beschreibt die deutsche Patentanmeldung DE 20 2004 035 348 A1 Haarfärbemittel, umfassend zwei voneinander getrennte Zubereitungen, wobei eine dieser Zubereitungen von einem wasserlöslichen Verpackungsmittel eingeschlossen ist.

Das europäische Patent EP 493 392 B1 offenbart Haarfärbe- und bleichmittel, die in Polyvinylalkohol-Verpackungen eingearbeitet werden, um die durch Pulverstaub verursachten Irritationen zu vermindern.

Die europäische Patentanmeldung EP 1 510 529 A1 beschreibt die Herstellung multimodaler Dispersionen von Vinylalkohol/Vinylacetat-Copolymeren.

Die WO 91/01127 A1 beschreibt ein Verfahren zum Bleichen von Haaren, das die Verwendung einer Bleichverpackung, enthaltend eine wirksame Menge an Haarbleichmittel, die von einer wasserlöslichen Umhüllen umschlossen ist, umfasst.

Die US 3,892,905 offenbart Polyvinylalkohol/Polyvinylpyrrolidon-haltige Folien zur Verpackung von Pflanzenschutzmitteln.

Die im Stand der Technik offenbarten portionierten kosmetischen Formulierungen bieten zwar eine verbesserte Handhabbarkeit und eine Verringerung der Staubkontamination der verpackten kosmetischen Zubereitungen, jedoch weisen die in wasserlöslichen Foliensystemen verpackten Portionen den Nachteil auf, dass sie sich in Wasser nur langsam lösen. Die Aufgabe der vorliegenden Erfindung ist es daher, portionierte kosmetische Zubereitungen zum Bleichen keratinsicher Fasern bereitzustellen, die einfach, sicher und schnell handzuhaben sind, deren Handhabung also beispielsweise ohne Staubentwicklung erfolgen kann und dem Verbraucher in kurzer Zeit eine gebrauchsfertige Anwendungsmischung liefert.

Es wurde gefunden, dass die vorgenannten Probleme durch spezielle Verpackungsmittel gelöst werden kann. Diese Verpackungsmittel vermeiden nicht nur eine Staubentwicklung sondern ermöglichen zudem eine überraschend schnelle und rückstandsfreie Herstellung der haarkosmetischen Anwendungsmischung.

Ein erster Gegenstand der vorliegenden Erfindung ist ein kosmetisches Mittel zum Färben keratinischer Fasern, enthaltend mindestens zwei getrennt voneinander verpackte Zubereitungen (A) und (B), die unmittelbar vor der Anwendung zu einer Anwendungsmischung vermischt werden, wobei
- die Zubereitung (A) mindestens ein Färbemittel aus der Gruppe der Oxidationsfarbstoffvorprodukte und der direktziehenden Farbstoffe enthält,
- die Zubereitung (B) eine fließfähige Zusammensetzung ist, die Wasser und mindestens ein Oxidationsmittel enthält,
dadurch gekennzeichnet, dass die Zubereitung (A) in einer wasserlöslichen Folie verpackt ist, welche, bezogen auf ihr Gesamtgewicht, zu mindestens 60 Gew.-% aus einem Polymergemisch umfassend
a1) wasserlösliches Vinylalkohol/Vinylacetat-Copolymer a1) und
a2) mindestens ein gegebenenfalls modifiziertes wasserlösliches Polysaccharid, besteht, das eine multimodale Molekulargewichtsverteilung aufweist.

Die Molekulargewichtsverteilung des in der wasserlöslichen Folie enthaltenen Polymergemischs ist multimodal. Mit anderen Worten weist die Dichte der Häufigkeitsverteilung des Molekulargewichts mindestens zwei Modi (Maxima), beispielsweise zwei, drei, vier, fünf oder mehr Modi auf. Besonders bevorzugt ist eine bimodale Molekulargewichtsverteilung, da sich diese, wie eingangs beschrieben, einerseits sehr vorteilhaft auf die Produkteigenschaften erfindungsgemäßer kosmetischer Mittel auswirkt, andererseits einfacher zu realisieren ist als eine tri- oder mehrmodale Häufigkeitsverteilung.

Die bevorzugte bimodale Molekulargewichtsverteilung kann symmetrisch oder asymmetrisch sein.

In einer bevorzugten multimodalen, vorzugsweise bimodalen Molekulargewichtsverteilung, unterscheiden sich die Molekulargewichte mindestens zwei der Modi, bezogen auf das kleinste einem Modus zuordenbare Molekulargewicht, um 5% bis 120%, vorzugsweise um 10% bis 90% und insbesondere um 20% bis 60%

In einer weiteren bevorzugten multimodalen, vorzugsweise bimodalen Molekulargewichtsverteilung, unterscheidet sich die Häufigkeit des zwischen zwei Modi befindlichen Minimums von der Häufigkeit des kleinsten dieser beiden Modi (Modus mit der geringsten Häufigkeit), um 5% bis 80%, vorzugsweise 10% bis 60% und insbesondere 20% bis 40%, jeweils bezogen auf die Häufigkeit des kleinsten der beiden Modi.

Für die Anwendungseigenschaften erfindungsgemäßer Mittel, insbesondere die schnelle und rückstandsfreie Herstellung der haarkosmetischen Anwendungsmischung hat es sich als vorteilhaft erwiesen, wenn die wasserlösliche Folie, bezogen auf ihr Gesamtgewicht, zu mindestens 70 Gew.-%, vorzugsweise zu mindestens 80 Gew.-%, bevorzugt zu mindestens 90 Gew.-% und insbesondere zu mindestens 95 Gew.-% aus einem Polymergemisch besteht, das eine multimodale Molekulargewichtsverteilung aufweist. Wiederum ist eine bimodale Molekulargewichtsverteilung bevorzugt.

Als für die Produkteigenschaften vorteilhaft haben sich Polymergemische erwiesen, welche einen Polydispersitätsindex oberhalb 2,2, vorzugsweise oberhalb 3,0, und insbesondere oberhalb 4,6 aufweisen. Als Polydispersitätsindex wird dabei das Verhältnis von gewichts- und zahlenmittlerer Molmasse bezeichnet.

Das Gewichtsmittel oder die gewichtsmittlere Molmasse (Mₘᵢₜ) ist definiert als Mₘᵢₜ = ∑nᵢ Mᵢ² / ∑nᵢ Mᵢ
mit Mₘᵢₜ = gewichtsmittlere Molmasse, Nᵢ = Zahl der Makromoleküle in der Probe mit genau i Repetiereinheiten und Mᵢ = Molmasse i.

Das Gewichtsmittel erhält man durch Methoden, die Größe und Gestalt eines Moleküls in Lösung berücksichtigen, z.B. statische Lichtstreuung, Röntgenkleinwinkelstreuung und Sedimentationsgleichgewichtsmessungen.

Das Zahlenmittel oder die zahlenmittlere Molmasse (Mₙ) ist definiert als Mₙ = ∑nᵢ Mᵢ² / ∑ nᵢ Mᵢ mit Mₙ = zahlenmittlere Molmasse, nᵢ = Zahl der Makromoleküle in der Probe mit genau i Repetiereinheiten und Mᵢ = Molmasse i.

Das Zahlenmittel lässt sich durch kolligative Verfahren wie z.B. Kryoskopie, Membran- oder Dampfdruckosmometrie bestimmen und - sofern die Anzahl der Endgruppen pro Molekül bekannt ist - durch Endgruppenbestimmung.

Wasserlösliche Folien, die nicht vollständig aus dem Polymergemisch mit der multimodalen Molekulargewichtsverteilung bestehen, können als weitere Inhaltsstoffe zusätzliche Wirk- oder Füllstoffe aber auch Lösungsmittel, insbesondere Wasser, enthalten.

Zur Gruppe der weiteren Wirkstoffe zählen dabei beispielsweise haarkosmetisch wirksame Bestandteile ebenso wie Materialien, welche die von dem Folienmaterial umschlossenen Inhaltsstoffe der Zubereitung (A) vor Zersetzung oder Desaktivierung durch Lichteinstrahlung schützen. Als besonders geeignet haben sich hier Antioxidantien, UV-Absorber und Fluoreszensfarbstoffe erwiesen.

Die wasserlösliche Folie weist, bezogen auf ihr Gesamtgewicht, vorzugsweise einen Wassergehalt von 3,0 bis 12 Gew.-%, bevorzugt von 4,0 bis 10 Gew.-% auf.

Die Dicke der zur Verpackung der Zubereitung (A) eingesetzten wasserlöslichen Folie beträgt bevorzugt 0,01 bis 0,1 mm, vorzugsweise von 0,01 bis 0,08 mm und insbesondere von 0,02 bis 0,06 mm.

In dem erfindungsgemäßen kosmetischen Mittel besteht das Polymergemisch bezogen auf sein Gesamtgewicht zu mindestens 60 Gew.-%, bevorzugt zu mindestens 80 Gew.-%, vorzugsweise zu mindestens 90 Gew.-%, besonders bevorzugt zu mindestens 95 Gew.-% aus einer Mischung umfassend
a1) wasserlösliches Vinylalkohol/Vinylacetat-Copolymer a1) und
a2) mindestens ein gegebenenfalls modifiziertes wasserlösliches Polysaccharid, vorzugsweise mindestens ein wasserlösliches Polysaccharid aus der Gruppe Methylcellulose, Carboxymethylcellulose, Ethylcellulose, Hydroxyethylcellulose, Hydroxypropylcellulose, Hydroxypropylmethylcellulose, Dextrin und Hydroxypropylstärke, besonders bevorzugt mindestens ein wasserlösliches Polysaccharid aus der Gruppe der Hydroxypropylstärken.

Der Polydispersitätsindex der vorgenannten Polymermischungen aus Vinylalkohol/VinylacetatCopolymere und Polysaccharid liegt vorzugsweise oberhalb 2,2, bevorzugt oberhalb 3,0 und insbesondere oberhalb 4,6, während das Vinylalkohol/Vinylacetat-Copolymer a1) in diesen Mischungen bevorzugt einen Polydispersitätsindex zwischen 1,8 und 2,3 aufweist.

Das Vinylalkohol/Vinylacetat-Copolymer a1) weist vorzugsweise einen Hydrolysegrad zwischen 84% und 90%, bevorzugt zwischen 85% und 89% und insbesondere zwischen 86% und 88% auf. Die Viskosität (20°C, 4 Gew.-%ige Lösung in Wasser, gemessen mit einem Brookfield LV Viscosimeter mit UL Adapter) des Vinylalkohol/Vinylacetat-Copolymer a1) liegt vorzugsweise zwischen 12 cP und 20 cP, bevorzugt zwischen 14 cP und 19 cP und insbesondere zwischen 16 cP und 18 cP.

Neben den zuvor beschriebenen wasserlöslichen Folien umfassen die erfindungsgemäßen kosmetischen Mittel die Zubereitungen (A) und (B). Für das Auflösungs- und Anwendungsverhalten dieser Mittel hat es sich als vorteilhaft erwiesen, wenn das Gewichtsverhältnis der Zubereitung (A) zur Zubereitung (B) 2:1 bis 1:3 beträgt.

Die Zubereitung (A) enthält als kennzeichnenden Bestandteil mindestens ein Färbemittel aus der Gruppe der Oxidationsfarbstoffvorprodukte und der direktziehenden Farbstoffe. Der Gewichtsanteil der Oxidationsfarbstoffvorprodukte und der direktziehenden Farbstoffe, vorzugsweise der Oxidationsfarbstoffvorprodukte, am Gesamtgewicht der Zubereitung (A) beträgt vorzugsweise 0,1 bis 10 Gew.-%, vorzugsweise 0,2 bis 8,0 Gew.-% und insbesondere 0,5 bis 6,0 Gew.-%.

In einer ersten bevorzugten Ausführungsform enthält die Zubereitung (A) mindestens ein oxidatives Färbemittel (Oxidationsfarbstoffvorprodukt). Unter oxidativen Färbemittel sind erfindungsgemäß haarfarbverändernde Mittel zu verstehen, die durch Oxidation von Oxidationsfarbstoffvorprodukten eine dauerhafte Färbung der Fasern bewirken. Unter der Bezeichnung Oxidationsfarbstoffvorprodukt werden sogenannte Entwicklerkomponenten und Kupplerkomponenten zusammengefasst. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Hinsichtlich der in der erfindungsgemäßen Zubereitung (A) einsetzbaren Farbstoffvorprodukte unterliegt die vorliegende Erfindung keinerlei Einschränkungen. Die erfindungsgemäße Zubereitung (A) kann als Farbstoffvorprodukte Oxidationsfarbstoffvorprodukte vom Entwickler- und/oder Kuppler-Typ, und Vorstufen naturanaloger Farbstoffe, wie Indol- und Indolin-Derivate, sowie Mischungen von Vertretern dieser Gruppen enthalten.

Im Rahmen einer ersten bevorzugten Ausführungsform der vorliegenden Erfindung enthalten die erfindungsgemäße Zubereitung (A) mindestens ein Oxidationsfarbstoffvorprodukt vom Entwickler- und/oder Kupplertyp.

Es kann erfindungsgemäß bevorzugt sein, als Entwicklerkomponente ein p-Phenylendiaminderivat oder eines seiner physiologisch verträglichen Salze einzusetzen. Besonders bevorzugte p-Phenylendiamine sind ausgewählt aus p-Phenylendiamin, p-Toluylendiamin, 2-Chlor-p-phenylendiamin, N,N-Bis-(2-hydroxyethyl)-p-phenylendiamin und N-Phenyl-p-phenylendiamin, 2-(2-Hydroxyethyl)-p-phenylendiamin, N-(4-Amino-3-methylphenyl)-N-[3-(1H-imidazol-1-yl)propyl]amin sowie ihren physiologisch verträglichen Salzen. Besonders bevorzugte kosmetische Mittel sind dadurch gekennzeichnet, dass Zubereitung (A), mindestens ein Oxidationsfarbstoffvorprodukt vom Entwicklertyp, vorzugsweise mindestens eine Verbindung aus der Gruppe p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin und/oder deren physiologisch verträgliche Salze enthält.

In einer weiteren bevorzugten Ausführungsform werden als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten sind insbesondere: N,N'-Bis-(2-hydroxyethyl)-N,N'-bis-(4'-aminophenyl)-1,3-diamino-propan-2-ol und Bis-(2-hydroxy-5-aminophenyl)-methan und deren physiologisch verträgliche Salze.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, und 4-Amino-3-methylphenol sowie deren physiologisch verträgliche Salze. Ferner kann die Entwicklerkomponente ausgewählt sein aus o-Aminophenol und seinen Derivaten, wie beispielsweise 2-Amino-5-methylphenol und dessen physiologisch verträglichen Salzen. Besonders bevorzugte kosmetisches Mittel sind dadurch gekennzeichnet, dass Zubereitung (A), mindestens ein Oxidationsfarbstoffvorprodukt vom Entwicklertyp, vorzugsweise mindestens eine Verbindung aus der Gruppe Bis-(2-hydroxy-5-aminophenyl)methan, p-Aminophenol, 4-und Amino-3-methylphenol und/oder deren physiologisch verträgliche Salze enthält.

Schließlich kann die Entwicklerkomponente auch sein aus heterocyclischen Entwicklerkomponenten, wie beispielsweise den Pyridin-, Pyrimidin-, Pyrazol-, Pyrazolo-PyrimidinDerivaten und ihren physiologisch verträglichen Salzen ausgewählt sein. Bevorzugte PyrimidinDerivate sind insbesondere 2,4,5,6-Tetraaminopyrimidin und 4-Hydroxy-2,5,6-triaminopyrimidin und deren physiologisch verträgliche Salze. Ein bevorzugtes Pyrazol-Derivat ist 4,5-Diamino-1-(2-hydroxyethyl)-pyrazol und dessen physiologisch verträgliche Salze. Besonders bevorzugte kosmetisches Mittel sind dadurch gekennzeichnet, dass Zubereitung (A), mindestens ein Oxidationsfarbstoffvorprodukt vom Entwicklertyp, vorzugsweise mindestens eine Verbindung aus der Gruppe 4,5-Diamino-1-(2-hydroxyethyl)pyrazol, 2,4,5,6-Tetraaminopyrimidin, 4-Hydroxy-2,5,6-triaminopyrimidin und/oder deren physiologisch verträgliche Salze enthält.

In einer weiteren bevorzugten Ausführungsform enthalten die Zubereitungen B mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5- und 2,7-Dihydroxynaphthalin, 1-Acetoxy-2-methoxynaphthalin, Resorcin, 4-Chlor-resorcin und 2-Amino-3-hydroxypyridin und deren physiologisch verträgliche Salze.

Erfindungsgemäß bevorzugte Kupplerkomponenten sind
(A) m-Aminophenol und dessen Derivate wie beispielsweise 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,
(B) o-Aminophenol und dessen Derivate, beispielsweise 2-Amino-5-ethylphenol,
(C) m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol, 2-({3-[(2-Hydroxyethyl)amino]-4-methoxy-5-methylphenyl}amino)ethanol und 2-({3-[(2-Hydroxyethyl)amino]-2-methoxy-5-methylphenyl}amino)ethanol,
(D) o-Diaminobenzol und dessen Derivate,
(E) Di- beziehungsweise Trihydroxybenzolderivate wie beispielsweise 2-Methylresorcin und 1,2,4-Trihydroxybenzol,
(F) Pyridinderivate wie beispielsweise 3-Amino-2-methylamino-6-methoxypyridin, 2,6-Diaminopyridin, 2,6-Dihydroxy-3,4-dimethylpyridin, 2-Amino-3-hydroxypyridin und 3,5-Diamino-2,6-dimethoxypyridin,
(G) Naphthalinderivate wie beispielsweise 1-Naphthol und 2-Methyl-1-naphthol,
(H) Morpholinderivate wie beispielsweise 6-Hydroxybenzomorpholin,
(I) Chinoxalinderivate,
(J) Pyrazolderivate wie beispielsweise 1-Phenyl-3-methylpyrazol-5-on,
(K) Indolderivate wie beispielsweise 6-Hydroxyindol,
(L) Pyrimidinderivate oder
(M) Methylendioxybenzolderivate wie beispielsweise 1-(2'-Hydroxyethyl)-amino-3,4-methylendioxybenzol
sowie deren physiologisch verträgliche Salze.

Besonders bevorzugte alternative kosmetische Mittel sind dadurch gekennzeichnet, dass Zubereitung (A),
- mindestens ein Oxidationsfarbstoffvorprodukt vom Kupplertyp, vorzugsweise mindestens eine Verbindung aus der Gruppe 3-Aminophenol, 5-Amino-2-methylphenol, 3-Amino-2-chlor-6-methylphenol, 2,4-Dichlor-3-aminophenol und/oder deren physiologisch verträgliche Salze enthält;
- mindestens eine Verbindung aus der Gruppe 2-(2,4-Diaminophenoxy)ethanol, 1,3-Bis(2,4-diaminophenoxy)propan, 1-Methoxy-2-amino-4-(2-hydroxyethylamino)benzol, 2,6-Bis(2'-hydroxyethylamino)-1-methylbenzol und/oder deren physiologisch verträgliche Salze enthält;
- mindestens eine Verbindung aus der Gruppe Resorcin, 2-Methylresorcin und 4-Chlorresorcin enthält;
- mindestens eine Verbindung aus der Gruppe 2-Amino-3-hydroxypyridin, 3-Amino-2-methylamino-6-methoxypyridin, 2,6-Dihydroxy-3,4-dimethylpyridin und/oder deren physiologisch verträgliche Salze enthält;
- mindestens eine Verbindung aus der Gruppe 2-Naphthol und 2,7-Dihydroxynaphthalin enthält.

Erfindungsgemäß ganz besonders bevorzugte Kupplerkomponenten sind 1-Naphthol, 1,5- und 2,7-Dihydroxynaphthalin, 5-Amino-2-methylphenol, 2-Amino-3-hydroxypyridin, Resorcin, 4-Chlorresorcin, 2-Methylresorcin und 2,6-Dihydroxy-3,4-dimethylpyridin und deren physiologisch verträgliche Salze.

Die Zubereitung (A) enthält sowohl die Entwicklerkomponenten als auch die Kupplerkomponenten bevorzugt in einer Menge von 0,1 bis 5,0 Gew.-%, vorzugsweise 0,2 bis 5,0 Gew.-% und insbesondere 0,5 bis 5,0 Gew.-%., jeweils bezogen auf das Gesamtgewicht der Zubereitung (A). Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

In einer weiteren Ausführungsform der vorliegenden Erfindung enthält die Zubereitung (A) als Oxidationsfarbstoffvorprodukt mindestens eine Vorstufe eines naturanalogen Farbstoffs. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Besonders bevorzugte Derivate des Indolins sind das 5,6-Dihydroxyindolin und das 2,3-Dioxoindolin (Isatin) und deren physiologisch verträglichen Salze. Ein besonders bevorzugtes Derivat des Indols ist das 5,6-Dihydroxyindol und dessen physiologisch verträglichen Salze.

Neben den Oxidationsfarbstoffvorprodukten oder alternativ zu diesen Färbemitteln kann die Zubereitung (A) auch direktziehende Farbstoffe enthalten. In einer weiteren bevorzugten Ausführungsform enthält die Zubereitung (A) mindestens einen direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen oder den Indophenolen und deren physiologisch verträglichen Salze.

Als anionische direktziehende Farbstoffe eignen sich insbesondere 2,4-Dinitro-1-naphthol-7-sulfonsäure-dinatriumsalz (C.I. 10,316; Acid Yellow 1; Food Yellow No. 1), 2-(Indan-1,3-dion-2-yl)chinolin-x,x-sulfonsäure (Gemisch aus Mono- und Disulfonsäure) (C.I. 47,005; D&C Yellow No. 10; Food Yellow No. 13; Acid Yellow 3, Yellow 10), 5-Hydroxy-1-(4-sulfophenyl)-4-[(4-sulfophenyl)-azo]pyrazol-3-carbonsäure-trinatriumsalz (C.I. 19,140; Food Yellow No. 4; Acid Yellow 23), 3-[(4-Phenylamino)phenyl]azobezolsulfonsäuresäure-natriumsalz (C.I. 13,065; Ki406; Acid Yellow 36), 4-[(2-Hydroxynaphth-1-yl)azo]-benzolsulfonsäure-natriumsalz (C.I. 15,510; Acid Orange 7), 6-Hydroxy-5-[(4-sulfonaphth-1-yl)azo]-2,4-naphthalin-disulfonsäure-trinatriumsalz (C.I. 16,255; Ponceau 4R; Acid Red 18), 8-Amino-1-hydroxy-2-(phenylazo)-3,6-naphthalin-disulfonsäure-dinatriumsalz (C.I. 17,200; Acid Red 33; Red 33), N-[6-(Diethylamino)-9-(2,4-disulfophenyl)-3H-xanthen-3-yliden]-N-ethylethanammonium-hydroxid, inneres Salz, Natriumsalz (C.I. 45,100; Acid Red 52), 2',4',5',7'-Tetrabrom-4,5,6,7-tetrachlor-3',6'- dihydroxyspiro[isobenzofuran-1(3H),9'[9H]xanthen]-3-on-dinatriumsalz (C.I. 45,410; Acid Red 92), 3-Hydroxy-4-[(4-methyl-2-sulfonphenyl)azo]-2-naphthalincarbonsäure-calciumsalz (C.I. 15,850:1; Pigment Red 57:1), 1,4-Bis[(2-sulfo-4-methylphenyl)amino]-9,10-anthrachinon-dinatriumsalz (C.I. 61,570; Acid Green 25), Bis[4-(dimethylamino)phenyl]-(3,7-disulfo-2-hydroxynaphth-1-yl)carbenium-inneres Salz, Natriumsalz (C.I. 44,090; Food Green No. 4; Acid Green 50), N-[4-[(2,4-Disulfophenyl)[4-[ethyl(phenylmethyl)amino)phenyl]methylen]-2,5-cyclohexadien-1-yliden]-N-ethylbenzolmethanaminium-hydroxid, inneres Salz, Natriumsalz (C.I. 42,080; Acid Blue 7), (2-Sulfophenyl)di[4-(ethyl((4-sulfophenyl)methyl)amino)phenyl]-carbenium-dinatriumsalz Betain (C.I. 42,090; Acid Blue 9; FD&C Blue No. 1), 1-Amino-4-(cyclohexylamino)-9,10-anthrachinon-2-sulfonsäure-natriumsalz (C.I. 62045; Acid Blue 62), 1-Hydroxy-4-[(4-methyl-2- sulfophenyl)amino]-9,10-anthrachinon-natriumsalz (C.I. 60,730; D&C Violett No. 2; Acid Violet 43), 5-Amino-4-hydroxy-6-[(4-nitrophenyl)-azo]-3-(phenylazo)-2,7-naphthalin-disulfonsäure-dinatriumsalz (C.I. 20,470; Acid Black 1), 3-Hydroxy-4-[(2-hydroxynaphth-1-yl)azo]-7-nitro-1-naphthalin-sulfonsäure-chromkomplex (3:2) (C.I. 15,711; Acid Black 52), 3',3",4,5,5',5",6,7-Octabromphenolsulfonphthalein (Tetrabromphenolblau).

Bevorzugte anionische direktziehende Farbstoffe sind die unter den internationalen Bezeichnungen bzw. Handelsnamen Acid Yellow 1, Acid Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Als kationische direktziehende Farbstoffe eignen sich insbesondere Di[4-(diethylamino)phenyl][4-(ethylamino)naphthyl]carbenium-chlorid (C.I. 42,595; Basic Blue 7), Di[4-(dimethylamino)phenyl][4-(phenylamino)naphthyl] carbenium-chlorid (C.I. 44,045; Basic Blue 26), 8-Amino-2-brom-5-hydroxy-4-imino-6-[(3-(trimethylammonio)phenyl)amino]-1(4H)-naphthalinon-chlorid (C.I. 56,059; Basic Blue No. 99), Tri(4-amino-3-methylphenyl)carbenium-chlorid (C.I. 42,520; Basic Violet 2), Di(4-aminophenyl)(4-amino-3- methylphenyl)carbeniumchlorid (C.I. 42,510 Basic Violet 14), 1-[(4-Aminophenyl)azo]-7-(trimethylammonio)- 2-naphthol-chlorid (C.I. 12,250; Basic Brown 16), 1-[(4-Amino-3-nitrophenyl)azo]-7-(trimethylammonio)-2-naphthol-chlorid (C.I. 12,251; Basic Brown 17), 3-[(4-Amino-2,5- dimethoxyphenyl)azo]-N,N,N-trimethylbenzolaminium-chlorid (C.I. 12,605, Basic Orange 69), 2-[((4-Dimethylamino)phenyl)azo]-1,3-dimethyl-1H-imidazoliumchlorid (Basic Red 51), 2-Hydroxy-1-[(2-methoxyphenyl)azo]-7-(trimethylammonio)-naphthalin-chlorid (C.I. 12,245; Basic Red 76), 2-[4-Aminophenyl]azo]-1,3-dimethyl-1H-Imidazolium-chlorid (Basic Orange 31), 3-Methyl-1-phenyl-4-[(3-(trimethylammonio)phenyl)azo]-pyrazol-5-on-chlorid (C.I. 12,719; Basic Yellow 57), 1-Methyl-4-((methylphenylhydrazono)methyl)-pyridinium-methylsulfat (Basic Yellow 87), 1-(2-Morpholiniumpropylamino)-4-hydroxy-9,10-anthrachinon-methylsulfat, 4-Formyl-1-methylquinolonium-p-toluensulfonat und direktziehende Farbstoffe, die einen Heterocyclus enthalten, der mindestens ein quaternäres Stickstoffatom aufweist.

Als nichtionische direktziehende Farbstoffe eignen sich insbesondere nichtionische Nitro- und Chinonfarbstoffe und neutrale Azofarbstoffe.

Geeignete blaue Nitrofarbstoffe sind insbesondere 1,4-Bis[(2-hydroxyethyl)amino]-2-nitrobenzol (HC Violet BS), 1-(2-Hydroxyethyl)amino-2-nitro-4-[di(2-hydroxyethyl)amino]-benzol (HC Blue 2), 4-[Di(2-hydroxyethyl)amino]-1-[(2-methoxyethyl)amino]-2-nitrobenzol (HC Blue 11), 4-[Ethyl-(2-hydroxyethyl)-amino]-1-[(2-hydroxyethyl)amino]-2-nitrobenzol-hydrochlorid (HC Blue 12), 1-(2-Hydroxyethyl)amino-2-nitro-4-N-ethyl-N-(2-hydroxyethyl)aminobenzol (HC Blue 15), 1-Amino-3-methyl-4-[(2-hydroxyethyl)amino]-6-nitrobenzol (HC Violet 1), 1-(3-Hydroxypropylamino)-4-[di(2-hydroxyethyl)amino]-2-nitrobenzol (HC Violet 2).

Geeignete rote Nitrofarbstoffe sind insbesondere 1-Amino-4-[(2-hydroxyethyl)amino]-2-nitrobenzol (HC Red 7), 2-Amino-4,6-dinitrophenol (Pikraminsäure) und deren Salze, 1,4-Diamino-2-nitrobenzol (C.I. 76,070), 4-Amino-2-nitro-diphenylamin (HC Red 1), 1-Amino-4-[di(2-hydroxyethyl)amino]-2-nitrobenzol-hydrochlorid (HC Red 13), 1-Amino-4-[(2-hydroxyethyl)-amino]-5-chlor-2-nitrobenzol, 4-Amino-1-[(2-hydroxyethyl)amino]-2-nitrobenzol (HC Red 3), 4-[(2-Hydroxyethyl)-amino]-3-nitrotoluol, 4-Amino-3-nitrophenol, 4-[(2-Hydroxyethyl)-amino]-3-nitrophenol, 4-[(2-Nitrophenyl)amino]phenol (HC Orange 1), 1-[(2-Aminoethyl)amino]-4-(2-hydroxyethoxy)-2-nitrobenzol (HC Orange 2), 1-Amino-5-chlor-4-[(2,3-dihydroxypropyl)amino]-2-nitrobenzol (HC Red 10), 5-Chlor-1,4-[di(2,3-dihydroxypropyl)amino]-2-nitrobenzol (HC Red 11), 2-[(2- Hydroxyethyl)amino]-4,6-dinitrophenol und deren Salze, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol (HC Red BN), 1,2,3,4-Tetrahydro-6-nitrochinoxalin, 6-Hydroxy-5-((2-methoxy-5-methyl-4-sulfophenyl)azo)- 2-naphthalensulfonsäure (Curry Red).

Geeignete gelbe Nitrofarbstoffe sind insbesondere 1,2-Diamino-4-nitrobenzol (C.I. 76,020), 1-[(2-Hydroxyethyl)amino]-2-nitrobenzol (HC Yellow 2), 1-(2-Hydroxyethoxy)-2-[(2-hydroxyethyl)amino]-5-nitrobenzol (HC Yellow 4), 1-Amino-2-[(2-hydroxyethyl)amino]-5-nitrobenzol (HC Yellow 5), 4-[(2,3-Dihydroxypropyl)-amino]-3-nitro-1-trifluormethyl-benzol (HC Yellow 6), 2-[(2- Hydroxyethyl)amino]-1-methoxy-5-nitrobenzol, 2-Amino-4-nitrophenol, 1-(2-Hydroxyethoxy)-3-methylamino-4-nitrobenzol, 2,3-(Dihydroxypropoxy)-3-methylamino-4-nitrobenzol, 3-[(2-Aminoethyl)amino]-1-methoxy-4-nitrobenzol-hydrochlorid (HC Yellow 9), 1-Chlor-2,4-bis[(2-hydroxyethyl)amino]-5-nitrobenzol (HC Yellow 10), 2-[(2-Hydroxyethyl)amino]-5-nitrophenol (HC Yellow 11), 1-[(2'-Ureidoethyl)amino]-4-nitrobenzol, 1-Amino-4-[(2-aminoethyl)amino]-5-methyl-2-nitrobenzol, 4-[(2-Hydroxyethyl)amino]-3-nitro-1-methylbenzol, 1-Chlor-4-[(2-hydroxyethyl)amino]-3-nitrobenzol (HC Yellow 12), 4-[(2-Hydroxyethyl)amino]-3-nitro-1-trifluormethyl-benzol (HC Yellow 13).

Geeignete Chinonfarbstoffe sind insbesondere 1-[(2-Hydroxyethyl)amino]-4-methylamino-9,10-anthrachinon (C.I. 61,505, Disperse Blue 3), Mischungen aus 1,4-bis[(2-hydroxyethyl)amino]anthra-9,10-quinon mit 1-[(2-hydroxyethyl)amino]-4-[(3-hydroxypropyl)amino]anthra-9,10-quinon und 1,4-bis[(3-hydroxypropyl)amino]anthra-9,10-quinone (Disperse Blue 377), 1,4-Diamino-9,10-anthrachinon (C.I. 61,100, Disperse Violet 1), 1-Amino-4-(methylamino)-9,10-anthrachinon (C.I. 61,105, Disperse Violet 4, Solvent Violet No. 12), 2-Hydroxy-1,4-naphthochinon (Lawsone, C.I. 75,480, Natural Orange 6), 1,4-bis[(2,3-dihydroxypropyl)amino]-9,10-anthracenedion (HC Blue 14).

Geeignete neutrale Azofarbstoffe sind insbesondere 1-[Di(2-hydroxyethyl)amino]-3-methyl-4-[(4-nitrophenyl)azo]-benzol (C.I. 11,210, Disperse Red 17), 1-[Di(2-hydroxyethyl)amino]-4-[(4-nitrophenyl)azo]-benzol (Disperse Black 9), 4-[(4- Aminophenyl)azo]-1-[di(2-hydroxyethyl)amino]-3-methylbenzol (HC Yellow 7), 2,6-Diamino-3-[(pyridin-3-yl)azo]-pyridin, 4-[(4-Nitrophenyl)azo]-anilin (C.I. 11,005; Disperse Orange 3).

Die Zubereitung (A) liegt vorzugsweise in fester Form, beispielsweise in Form eines Pulvers, eines Granulats oder eines verpressten Körpers, beispielsweise in Form einer Tablette vor. Bevorzugte kosmetische Zubereitungen (A) liegen in Pulverform vor.

Die Zubereitung (A) wird vor der Anwendung auf dem Haar vorzugsweise mit der wässrigen Zubereitung (B) vermischt und in dieser aufgelöst. Kennzeichnender Bestandteil der wässrigen Zubereitung (B) ist mindestens ein Oxidationsmittel. In einer bevorzugten Ausführungsform handelt es sich bei der Zubereitung (B) um eine wässrige Wasserstoffperoxid-Lösung. Bevorzugt ist eine Zubereitung (B), die bezogen auf ihr Gewicht, 50 bis 98 Gew.-%, vorzugsweise 60 bis 95 Gew.-%, weiter bevorzugt 70 bis 88 Gew.-% Wasser und 0,5 bis 20 Gew.-%, vorzugsweise 1 bis 15 Gew.-%, besonders bevorzugt 2 bis 12 Gew.-% Wasserstoffperoxid, berechnet als 100%iges H₂O₂, enthält.

Als weiteren Bestandteil enthält die Zubereitung (B) bevorzugt Emulgatoren bzw. oberflächenaktive Mittel.

Eine erste Gruppe bevorzugter sind die anionischen Tenside. Anionische Tenside im Sinne der Erfindung sind alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für solche anionischen Tenside sind, jeweils in Form der Natrium-, Kalium- und Ammoniumsowie der Mono, Di- und Trialkanolammoniumsalze mit 2 bis 4 C-Atomen in der Alkanolgruppe, lineare und verzweigte Fettsäuren mit 8 bis 30 C-Atomen (Seifen); Ethercarbonsäuren, insbesondere der Formel RO(CH₂CH₂O)ₓCH₂COOH, in der R eine lineare Alkylgruppe mit 8 bis 30 C-Atomen und x = 0 oder 1 bis 16 ist; Acylsarcoside; Acyltauride; Acylisethionate; Sulfobernsteinsäuremono- und - dialkylester sowie Sulfobernsteinsäuremono-alkylpolyoxyethylester; lineare Alkansulfonate; lineare α -Olefinsulfonate; Sulfonate ungesättigter Fettsäuren; α-Sulfofettsäuremethylester von Fettsäuren; Alkylsulfate und Alkylethersulfate, insbesondere der Formel RO(CH₂CH₂O)ₓSO₃H, in der R für eine lineare Alkylgruppe mit 8 bis 30 C-Atomen und x für 0 oder eine Zahl von 1 bis 12 steht; Gemische oberflächenaktiver Hydroxysulfonate; sulfatierte Hydroxyalkylpolyethylen- und/oder Hydroxyalkylenpropylenglykolether; Ester der Weinsäure und Zitronensäure mit Alkoholen; Alkyl- und/oder Alkenyletherphosphate der Formel RO(C₂H₄O)ₓP(=O)(OH)(OR'), worin R für einen aliphatischen, gegebenenfalls ungesättigten Kohlenwasserstoffrest mit 8 bis 30 Kohlenstoffatomen, R' für Wasserstoff, einen Rest (CH₂CH₂O)_{y}R und x und y unabhängig voneinander für eine Zahl von 1 bis 10 steht; sulfatierte Fettsäurealkylenglykolester der Formel RC(O)O(alkO)ₙSO₃H, in der R für einen linearen oder verzweigten, aliphatischen, gesättigten und/oder ungesättigten Alkylrest mit 6 bis 22 C-Atomen, alk für CH₂CH₂, CHCH₃CH₂ und/oder CH₂CHCH₃ und n für eine Zahl von 0,5 bis 5 steht; sowie Monoglyceridsulfate und Monoglyceridethersulfate. Besonders bevorzugt

Erfindungsgemäß bevorzugte kosmetische Mittel sind dadurch gekennzeichnet, dass diese zusätzlich mindestens ein anionisches Tensid enthalten. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Besonders bevorzugt sind C₈-C₂₀-Alkylsulfate, insbesondere Natriumcetearylsulfat und Natriumlaurylsulfat, sowie C₈-C₂₀-Alkylethersulfate mit 2 bis 12, vorzugsweise 2 bis 4 Ethylenoxidgruppen, insbesondere Natriumlaurylethersulfat (INCI: Sodium Laureth Sulfate). Der Gewichtsanteil des anionischen Tensids am Gesamtgewicht der Zubereitung (B) beträgt vorzugsweise 0,1 bis 8,0 Gew.-%, bevorzugt 0,1 bis 4,0 Gew.-% und insbesondere 0,1 bis 2,0 Gew.-%.

Bevorzugte Emulgatoren sind weiterhin PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil oder PEG-40 Hydrogenated Castor Oil. Erfindungsgemäß bevorzugt ist die Verwendung von PEG-40 Hydrogenated Castor Oil. Diese sind bevorzugt in einer Menge von 0,05 bis 1,5 Gew.-% enthalten, bevorzugter 0,1 bis 1,0 Gew.-%, ebenfalls bevorzugt 0,2 bis 0, 8 Gew.-% oder 0,3 bis 0,6 Gew.-%.

Um die Anwendungsmischung aus Zubereitung (A) und (B) sauber und lokal begrenzt applizieren zu können, hat sich eine höhere Viskosität des Mittels als vorteilhaft erwiesen. Dazu ist vorteilhaft, wenn das Mittel nicht als Paste, zähe Creme oder eingedicktes Gel vorliegt, sondern über eine ausreichende Fließfähigkeit verfügt. Weiterhin muss das anwendungsbereite Mittel zwar über rheologische Eigenschaften verfügen, die ein Auftragen auf die zu bleichenden Fasern erlauben, aber gleichzeitig ein Verlaufen oder Ausfließen des Mittels vom Wirkort während der Anwendungsdauer verhindern. Bevorzugt besitzen die Anwendungsmischungen daher eine Viskosität von 5 bis 100 Pa·s, bevorzugt von 10 bis 50 Pa·s, insbesondere von 10 bis 20 Pa·s und insbesondere bevorzugt von 10 bis 16 Pa·s (Brookfield, 22°C, Spindel #5, 4 rpm). Hierzu enthalten bevorzugte Zubereitungen (B) mindestens ein Verdickungsmittel und/oder mindestens einen Gelbildner. Entsprechende erfindungsgemäße Verfahren, bei denen die Zubereitung (B), zusätzlich mindestens ein Verdickungsmittel und/oder mindestens einen Gelbildner enthält, sind erfindungsgemäß bevorzugt. Als Verdickungsmittel bzw. Gelbildner eignen sich anorganische wie organische Substanzen.

Das Verdickungsmittel kann beispielsweise ausgewählt werden unter den unter folgenden INCI-Bezeichnungen bekannten polymeren Verdickungsmitteln: Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium Acryloyldimethyltaurate/VP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carbomer, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/Isophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxystearamide MEA, Isobutylene/Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates/Vinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Yeast Beta-Glucan, Yeast Polysaccharides, Zea Mays (Corn) Starch.

Besonders bevorzugt wird der polymere Verdicker ausgewählt unter polymeren, anionischen, amphiphilen Verdickern, besonders bevorzugt unter solchen mit den INCI-Bezeichnungen Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates / Palmeth-20 Acrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrylates/Vinyl Isodecanoate Crosspolymer, Acrylates / Steareth-20 Methacrylate Crosspolymer.

Die polymeren Verdickungsmittel sind bevorzugt in einer Menge von 0,5 bis 20 Gew.-% insbesondere von 0,5 bis 10 Gew.-% in der Zubereitung (B) enthalten.

Als besonders vorteilhaft hat es sich jedoch erwiesen, die Viskosität der durch Vermischen der Zubereitungen (A) und (B) erhältlichen Anwendungsmischung durch die Auswahl eines geeigneten Polymergemisches für die wasserlösliche Folie einzustellen. Die Viskosität der Anwendungsmischung und deren Anwendungseigenschaften und Bleichwirkung kann dabei sowohl durch die chemische Natur des Polymergemisches als auch durch die Menge des zur Verpackung eingesetzten Polymergemisches vorteilhaft beeinflusst. Bevorzugte kosmetische Mittel sind daher dadurch gekennzeichnet, dass der Gewichtsanteil des Polymergemisches mit der multimodalen Molekulargewichtsverteilung am Gesamtgewicht der Zubereitungen (A) und (B) einschließlich der wasserlöslichen Folie 1 bis 15 Gew.-%, vorzugsweise 2 bis 10 Gew.-% und insbesondere 3 bis 8 Gew.-.% beträgt.

Ferner kann die Zubereitung (B) weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Lösungsmittel und -vermittler wie Ethanol, Isopropanol, Ethylenglykol, Propylenglykol, Glycerin und Diethylenglykol,
- Konsistenzgeber wie Zuckerester, Polyolester oder Polyolalkylether,
- Stabilisierungsmittel für Wassserstoffperoxid, beispielsweise Komplexbildner wie EDTA, NTA, β -Alanindiessigsäure und Phosphonsäuren,
enthalten.

Für die Anwendungseigenschaften erfindungsgemäßer kosmetischer Mittel hat es sich als vorteilhaft erwiesen, den Gewichtsanteil hydrophober Bestandteile an der Zubereitung (B) weitest möglich zu reduzieren. Bevorzugte kosmetische Mittel sind daher dadurch gekennzeichnet, dass Zubereitung (B), bezogen auf ihr Gewicht, weniger als 20 Gew.-%, vorzugsweise weniger als 10 Gew.-% und insbesondere weniger als 5,0 Gew.-% Fettstoffe enthält. Zur Gruppe der Fettstoffe werden erfindungsgemäß solche Verbindungen gezählt, die bei 20°C zu weniger als 1 g in 100 g Wasser löslich sind. Hierzu zählen beispielsweise Wachse wie Candelillawachs, Carnaubawachs oder Bienenwachs, Sheabutter, Kokosfett, C₁₂ bis C₂₀- Fettsäuren (insbesondere Palmitinsäure, Stearinsäure), Silikone und Paraffine.

Wie eingangs ausgeführt, eignen sich erfindungsgemäße kosmetische Mittel insbesondere zur Herstellung haarbleichender Zubereitungen. Ein weiterer Gegenstand der vorliegenden Anmeldung ist daher ein Verfahren zur Farbveränderung keratinischer Fasern, bei dem mindestens zwei getrennt voneinander verpackte Zubereitungen (A) und (B) zu einer Anwendungsmischung vermischt werden indem die in einer wasserlöslichen Folie verpackte Zubereitung (A) unter Auflösung der wasserlöslichen Folie in die Zubereitung (B) eingetragen wird, wobei
- die Zubereitung (A), bezogen auf ihr Gesamtgewicht, 10 bis 99 Gew.-% Peroxidisulfat(e) enthält
- die Zubereitung (B) eine fließfähige Zusammensetzung ist, die Wasser und mindestens ein Oxidationsmittel enthält,
dadurch gekennzeichnet, dass die Zubereitung (A) in einer wasserlöslichen Folie verpackt ist, welche, bezogen auf ihr Gesamtgewicht, zu mindestens 60 Gew.-% aus einem Polymergemisch umfassend
a1) wasserlösliches Vinylalkohol/Vinylacetat-Copolymer a1) und
a2) mindestens ein gegebenenfalls modifiziertes wasserlösliches Polysaccharid, besteht, das eine multimodale Molekulargewichtsverteilung aufweist.

Die erfindungsgemäßen Zusammensetzungen, Verwendungen und Verfahren und einige ihrer bevorzugten Ausführungsformen sind durch die folgenden Punkte gekennzeichnet:
1. Kosmetisches Mittel zum Färben keratinischer Fasern, enthaltend mindestens zwei getrennt voneinander verpackte Zubereitungen (A) und (B), die unmittelbar vor der Anwendung zu einer Anwendungsmischung vermischt werden, wobei
   - die Zubereitung (A) mindestens ein Färbemittel aus der Gruppe der Oxidationsfarbstoffvorprodukte und der direktziehenden Farbstoffe enthält,
   - die Zubereitung (B) eine fließfähige Zusammensetzung ist, die Wasser und mindestens ein Oxidationsmittel enthält,
   dadurch gekennzeichnet, dass die Zubereitung (A) in einer wasserlöslichen Folie verpackt ist, welche, bezogen auf ihr Gesamtgewicht, zu mindestens 60 Gew.-% aus einem Polymergemisch umfassend
   a1) wasserlösliches Vinylalkohol/Vinylacetat-Copolymer a1) und
   a2) mindestens ein gegebenenfalls modifiziertes wasserlösliches Polysaccharid, besteht, das eine multimodale Molekulargewichtsverteilung aufweist.
2. Kosmetisches Mittel nach Punkt 1, dadurch gekennzeichnet, dass die wasserlösliche Folie, bezogen auf ihr Gesamtgewicht, zu mindestens 70 Gew.-%, vorzugsweise zu mindestens 80 Gew.-%, bevorzugt zu mindestens 90 Gew.-% und insbesondere zu mindestens 95 Gew.-% aus einem Polymergemisch besteht, das eine multimodale Molekulargewichtsverteilung aufweist.
3. Kosmetisches Mittel nach einem der vorherigen Punkte, dadurch gekennzeichnet, dass das Polymergemisch bezogen auf sein Gesamtgewicht zu mindestens 60 Gew.-%, bevorzugt zu mindestens 80 Gew.-%, vorzugsweise zu mindestens 90 Gew.-%, besonders bevorzugt zu mindestens 95 Gew.-% aus einer Mischung umfassend
   a1) wasserlösliches Vinylalkohol/Vinylacetat-Copolymer a1) und
   a2) mindestens ein gegebenenfalls modifiziertes wasserlösliches Polysaccharid, vorzugsweise mindestens ein wasserlösliches Polysaccharid aus der Gruppe Methylcellulose, Carboxymethylcellulose, Ethylcellulose, Hydroxyethylcellulose, Hydroxypropylcellulose, Hydroxypropylmethylcellulose, Dextrin und Hydroxypropylstärke, besonders bevorzugt mindestens ein wasserlösliches Polysaccharid aus der Gruppe der Hydroxypropylstärken
   besteht.
4. Kosmetisches Mittel nach einem der vorherigen Punkte, dadurch gekennzeichnet, dass das Polymergemisch einen Polydispersitätsindex oberhalb 2,2, vorzugsweise oberhalb 3,0, insbesondere oberhalb 4,6 aufweist.
5. Kosmetisches Mittel nach einem der vorherigen Punkte, dadurch gekennzeichnet, dass das Vinylalkohol/Vinylacetat-Copolymer a1) einen Polydispersitätsindex zwischen 1,8 und 2,3 aufweist.
6. Kosmetisches Mittel nach einem der vorherigen Punkte, dadurch gekennzeichnet, dass das Vinylalkohol/Vinylacetat-Copolymer a1) einen Hydrolysegrad zwischen 84% und 90%, vorzugsweise zwischen 85% und 89% und insbesondere zwischen 86% und 88% aufweist.
7. Kosmetisches Mittel nach einem der vorherigen Punkte, dadurch gekennzeichnet, dass das Vinylalkohol/Vinylacetat-Copolymer a1) eine Viskosität (20°C, 4 Gew.-%ige Lösung in Wasser, gemessen mit einem Brookfield LV Viscosimeter mit UL Adapter) zwischen 12 cP und 20 cP, vorzugsweise zwischen 14 cP und 19 cP und insbesondere zwischen 16 cP und 18 cP aufweist.
8. Kosmetisches Mittel nach einem der vorherigen Punkte, dadurch gekennzeichnet, dass die wasserlösliche Folie, bezogen auf ihr Gesamtgewicht, einen Wassergehalt von 3,0 bis 12 Gew.-%, vorzugsweise von 4,0 bis 10 Gew.-% aufweist.
9. Kosmetisches Mittel nach einem der vorherigen Punkte, dadurch gekennzeichnet, dass die wasserlösliche Folie eine Dicke von 0,01 bis 0,1 mm, vorzugsweise von 0,01 bis 0,08 mm und insbesondere von 0,02 bis 0,06 mm aufweist.
10. Kosmetisches Mittel nach einem der vorherigen Punkte, dadurch gekennzeichnet, dass das Gewichtsverhältnis der Zubereitung (A) zur Zubereitung (B) 2:1 bis 1:3 beträgt.
11. Kosmetisches Mittel nach einem der vorherigen Punkte, dadurch gekennzeichnet, dass Zubereitung (A), bezogen auf ihr Gewicht, 0,1 bis 10 Gew.-%, vorzugsweise 0,2 bis 8,0 Gew.-% und insbesondere 0,5 bis 6,0 Gew.-% Färbemittel aus der Gruppe der Oxidationsfarbstoffvorprodukte und der direktziehenden Farbstoffe, vorzugsweise der Oxidationsfarbstoffvorprodukte enthält.
12. Kosmetisches Mittel nach einem der vorherigen Punkte, dadurch gekennzeichnet, dass Zubereitung (A), mindestens ein Oxidationsfarbstoffvorprodukt vom Entwicklertyp, vorzugsweise mindestens eine Verbindung aus der Gruppe p-Toluylendiamin, 2-(2-Hydroxyethyl)-p-phenylendiamin, N,N-Bis-(2-hydroxyethyl)-p-phenylendiamin, 2-Methoxymethyl-p-phenylendiamin und/oder deren physiologisch verträgliche Salze enthält.
13. Kosmetisches Mittel nach einem der vorherigen Punkte, dadurch gekennzeichnet, dass Zubereitung (A), mindestens ein Oxidationsfarbstoffvorprodukt vom Entwicklertyp, vorzugsweise mindestens eine Verbindung aus der Gruppe Bis-(2-hydroxy-5-aminophenyl)methan, p-Aminophenol, 4-und Amino-3-methylphenol und/oder deren physiologisch verträgliche Salze enthält.
14. Kosmetisches Mittel nach einem der vorherigen Punkte, dadurch gekennzeichnet, dass Zubereitung (A), mindestens ein Oxidationsfarbstoffvorprodukt vom Entwicklertyp, vorzugsweise mindestens eine Verbindung aus der Gruppe 4,5-Diamino-1-(2-hydroxyethyl)pyrazol, 2,4,5,6-Tetraaminopyrimidin, 4-Hydroxy-2,5,6-triaminopyrimidin und/oder deren physiologisch verträgliche Salze enthält.
15. Kosmetisches Mittel nach einem der vorherigen Punkte, dadurch gekennzeichnet, dass Zubereitung (A), mindestens ein Oxidationsfarbstoffvorprodukt vom Kupplertyp, vorzugsweise mindestens eine Verbindung aus der Gruppe 3-Aminophenol, 5-Amino-2-methylphenol, 3-Amino-2-chlor-6-methylphenol, 2,4-Dichlor-3-aminophenol und/oder deren physiologisch verträgliche Salze enthält.
16. Kosmetisches Mittel nach einem der vorherigen Punkte, dadurch gekennzeichnet, dass Zubereitung (A), mindestens ein Oxidationsfarbstoffvorprodukt vom Kupplertyp, vorzugsweise mindestens eine Verbindung aus der Gruppe 2-(2,4-Diaminophenoxy)ethanol, 1,3-Bis(2,4-diaminophenoxy)propan, 1-Methoxy-2-amino-4-(2-hydroxyethylamino)benzol, 2,6-Bis(2'-hydroxy-ethylamino)-1-methylbenzol und/oder deren physiologisch verträgliche Salze enthält.
17. Kosmetisches Mittel nach einem der vorherigen Punkte, dadurch gekennzeichnet, dass Zubereitung (A), mindestens ein Oxidationsfarbstoffvorprodukt vom Kupplertyp, vorzugsweise mindestens eine Verbindung aus der Gruppe Resorcin, 2-Methylresorcin und 4-Chlorresorcin enthält.
18. Kosmetisches Mittel nach einem der vorherigen Punkte, dadurch gekennzeichnet, dass Zubereitung (A), mindestens ein Oxidationsfarbstoffvorprodukt vom Kupplertyp, vorzugsweise mindestens eine Verbindung aus der Gruppe 2-Amino-3-hydroxypyridin, 3-Amino-2-methylamino-6-methoxypyridin, 2,6-Dihydroxy-3,4-dimethylpyridin und/oder deren physiologisch verträgliche Salze enthält.
19. Kosmetisches Mittel nach einem der vorherigen Punkte, dadurch gekennzeichnet, dass Zubereitung (A), mindestens ein Oxidationsfarbstoffvorprodukt vom Kupplertyp, vorzugsweise mindestens eine Verbindung aus der Gruppe 2-Naphthol und 2,7-Dihydroxynaphthalin enthält.
20. Kosmetisches Mittel nach einem der vorherigen Punkte, dadurch gekennzeichnet, dass Zubereitung (B), bezogen auf ihr Gewicht, 50 bis 98 Gew.-%, vorzugsweise 60 bis 95 Gew.-%, weiter bevorzugt 70 bis 88 Gew.-% Wasser und 0,5 bis 20 Gew.-%, vorzugsweise 1 bis 15 Gew.-%, besonders bevorzugt 2 bis 12 Gew.-% Wasserstoffperoxid, berechnet als 100%iges H₂O₂, enthält.
21. Kosmetisches Mittel nach einem der vorherigen Punkte, dadurch gekennzeichnet, dass Zubereitung (B), bezogen auf ihr Gewicht, weniger als 20 Gew.-%, vorzugsweise weniger als 10 Gew.-% und insbesondere weniger als 5,0 Gew.-% Fettstoffe enthält.
22.Verfahren zur Farbveränderung keratinischer Fasern, bei dem mindestens zwei getrennt voneinander verpackte Zubereitungen (A) und (B) zu einer Anwendungsmischung vermischt werden indem die in einer wasserlöslichen Folie verpackte Zubereitung (A) unter Auflösung der wasserlöslichen Folie in die Zubereitung (B) eingetragen wird, wobei
   - die Zubereitung (A) mindestens ein Färbemittel aus der Gruppe der Oxidationsfarbstoffvorprodukte und der direktziehenden Farbstoffe enthält
   - die Zubereitung (B) eine fließfähige Zusammensetzung ist, die Wasser und mindestens ein Oxidationsmittel enthält,
   dadurch gekennzeichnet, dass die Zubereitung (A) in einer wasserlöslichen Folie verpackt ist, welche, bezogen auf ihr Gesamtgewicht, zu mindestens 60 Gew.-% aus einem Polymergemisch umfassend
   a1) wasserlösliches Vinylalkohol/Vinylacetat-Copolymer a1) und
   a2) mindestens ein gegebenenfalls modifiziertes wasserlösliches Polysaccharid, besteht, das eine multimodale Molekulargewichtsverteilung aufweist.

## Patentansprüche

1. Kosmetisches Mittel zum Färben keratinischer Fasern, enthaltend mindestens zwei getrennt voneinander verpackte Zubereitungen (A) und (B), die unmittelbar vor der Anwendung zu einer Anwendungsmischung vermischt werden, wobei
- die Zubereitung (A) mindestens ein Färbemittel aus der Gruppe der Oxidationsfarbstoffvorprodukte und der direktziehenden Farbstoffe enthält
- die Zubereitung (B) eine fließfähige Zusammensetzung ist, die Wasser und mindestens ein Oxidationsmittel enthält,
**dadurch gekennzeichnet, dass** die Zubereitung (A) in einer wasserlöslichen Folie verpackt ist, welche, bezogen auf ihr Gesamtgewicht, zu mindestens 60 Gew.-% aus einem Polymergemisch, umfassend
a1) wasserlösliches Vinylalkohol/Vinylacetat-Copolymer a1) und
a2) mindestens ein gegebenenfalls modifiziertes wasserlösliches Polysaccharid, besteht, das eine multimodale Molekulargewichtsverteilung aufweist.

2. Kosmetisches Mittel nach Anspruch 1, **dadurch gekennzeichnet, dass** das Polymergemisch bezogen auf sein Gesamtgewicht zu mindestens 80 Gew.-%, vorzugsweise zu mindestens 90 Gew.-%, besonders bevorzugt zu mindestens 95 Gew.-% aus einer Mischung umfassend
a1) wasserlösliches Vinylalkohol/Vinylacetat-Copolymer a1) und
a2) mindestens ein gegebenenfalls modifiziertes wasserlösliches Polysaccharid, vorzugsweise mindestens ein wasserlösliches Polysaccharid aus der Gruppe Methylcellulose, Carboxymethylcellulose, Ethylcellulose, Hydroxyethylcellulose, Hydroxypropylcellulose, Hydroxypropylmethylcellulose, Dextrin und Hydroxypropylstärke, besonders bevorzugt mindestens ein wasserlösliches Polysaccharid aus der Gruppe der Hydroxypropylstärken
besteht.

3. Kosmetisches Mittel nach einem der vorherigen Ansprüche, **dadurch gekennzeichnet, dass** das Polymergemisch einen Polydispersitätsindex oberhalb 2,2, vorzugsweise oberhalb 3,0, insbesondere oberhalb 4,6 aufweist.

4. Kosmetisches Mittel nach einem der vorherigen Ansprüche, **dadurch gekennzeichnet, dass** das Vinylalkohol/Vinylacetat-Copolymer a1) einen Hydrolysegrad zwischen 84% und 90%, vorzugsweise zwischen 85% und 89% und insbesondere zwischen 86% und 88% aufweist.

5. Kosmetisches Mittel nach einem der vorherigen Ansprüche, **dadurch gekennzeichnet, dass** die wasserlösliche Folie eine Dicke von 0,01 bis 0,1 mm, vorzugsweise von 0,01 bis 0,08 mm und insbesondere von 0,02 bis 0,06 mm aufweist.

6. Kosmetisches Mittel nach einem der vorherigen Ansprüche, **dadurch gekennzeichnet, dass** das Gewichtsverhältnis der Zubereitung (A) zur Zubereitung (B) 2:1 bis 1:3 beträgt.

7. Kosmetisches Mittel nach einem der vorherigen Ansprüche, **dadurch gekennzeichnet, dass** Zubereitung (A), bezogen auf ihr Gewicht, 0,1 bis 10 Gew.-%, vorzugsweise 0,2 bis 8,0 Gew.-% und insbesondere 0,5 bis 6,0 Gew.-% Färbemittel aus der Gruppe der Oxidationsfarbstoffvorprodukte und der direktziehenden Farbstoffe, vorzugsweise der Oxidationsfarbstoffvorprodukte enthält.

8. Kosmetisches Mittel nach einem der vorherigen Ansprüche, **dadurch gekennzeichnet, dass** Zubereitung (B), bezogen auf ihr Gewicht, weniger als 20 Gew.-%, vorzugsweise weniger als 10 Gew.-% und insbesondere weniger als 5,0 Gew.-% Fettstoffe enthält.

9. Verfahren zur Farbveränderung keratinischer Fasern, bei dem mindestens zwei getrennt voneinander verpackte Zubereitungen (A) und (B) zu einer Anwendungsmischung vermischt werden indem die in einer wasserlöslichen Folie verpackte Zubereitung (A) unter Auflösung der wasserlöslichen Folie in die Zubereitung (B) eingetragen wird, wobei
- die Zubereitung (A) mindestens ein Färbemittel aus der Gruppe der Oxidationsfarbstoffvorprodukte und der direktziehenden Farbstoffe enthält
- die Zubereitung (B) eine fließfähige Zusammensetzung ist, die Wasser und mindestens ein Oxidationsmittel enthält,
**dadurch gekennzeichnet, dass** die Zubereitung (A) in einer wasserlöslichen Folie verpackt ist, welche, bezogen auf ihr Gesamtgewicht, zu mindestens 60 Gew.-% aus einem Polymergemisch, umfassend
a1) wasserlösliches Vinylalkohol/Vinylacetat-Copolymer a1) und
a2) mindestens ein gegebenenfalls modifiziertes wasserlösliches Polysaccharid, besteht, das eine multimodale Molekulargewichtsverteilung aufweist.

## Claims

1. A cosmetic agent for coloring keratin fibers, containing at least two preparations (A) and (B) which are packaged separately from one another and which are mixed immediately before use to form an application mixture,
- the preparation (A) containing at least one colorant from the group of oxidation dye precursors and direct dyes
- the preparation (B) being a flowable composition containing water and at least one oxidizing agent,
**characterized in that** the preparation (A) is packaged in a water-soluble film, at least 60 wt.% of which, based on the total weight thereof, consists of a polymer mixture comprising
a1) a water-soluble vinyl alcohol/vinyl acetate copolymer a1) and
a2) at least one optionally modified water-soluble polysaccharide, which polymer mixture has a multimodal molecular weight distribution.

2. The cosmetic agent according to claim 1, **characterized in that** at least 80 wt.% of the polymer mixture, preferably at least 90 wt.%, particularly preferably at least 95 wt.%, based on the total weight thereof, consists of a mixture comprising
a1) a water-soluble vinyl alcohol/vinyl acetate copolymer a1) and
a2) at least one optionally modified water-soluble polysaccharide, preferably at least one water-soluble polysaccharide from the group of methyl cellulose, carboxymethyl cellulose, ethyl cellulose, hydroxyethyl cellulose, hydroxypropyl cellulose, hydroxypropyl methyl cellulose, dextrin and hydroxypropyl starch, particularly preferably at least one water-soluble polysaccharide from the group of hydroxypropyl starches.

3. The cosmetic agent according to one of the preceding claims, **characterized in that** the polymer mixture has a polydispersity index above 2.2, preferably above 3.0, in particular above 4.6.

4. The cosmetic agent according to one of the preceding claims, **characterized in that** the vinyl alcohol/vinyl acetate copolymer a1) has a degree of hydrolysis between 84% and 90%, preferably between 85% and 89%, and in particular between 86% and 88%.

5. The cosmetic agent according to one of the preceding claims, **characterized in that** the water-soluble film has a thickness of from 0.01 to 0.1 mm, preferably from 0.01 to 0.08 mm and in particular from 0.02 to 0.06 mm.

6. The cosmetic agent according to one of the preceding claims, **characterized in that** the weight ratio of preparation (A) to preparation (B) is from 2:1 to 1:3.

7. The cosmetic agent according to one of the preceding claims, **characterized in that** preparation (A) contains, based on the weight thereof, 0.1 to 10 wt.%, preferably 0.2 to 8.0 wt.% and in particular 0.5 to 6.0 wt.% of colorants from the group of oxidation dye precursors and direct dyes, preferably the oxidation dye precursors.

8. The cosmetic agent according to one of the preceding claims, **characterized in that** preparation (B) contains, based on the weight thereof, less than 20 wt.%, preferably less than 10 wt.% and in particular less than 5.0 wt.% of fatty substances.

9. A method for changing the color of keratin fibers, in which at least two separately packaged preparations (A) and (B) are mixed to form an application mixture by the preparation (A) which is packaged in a water-soluble film being introduced into the preparation (B) when the water-soluble film dissolves,
- the preparation (A) containing at least one colorant from the group of oxidation dye precursors and direct dyes
- the preparation (B) being a flowable composition containing water and at least one oxidizing agent,
**characterized in that** the preparation (A) is packaged in a water-soluble film, at least 60 wt.% of which, based on the total weight thereof, consists of a polymer mixture comprising
a1) a water-soluble vinyl alcohol/vinyl acetate copolymer a1) and
a2) at least one optionally modified water-soluble polysaccharide, which polymer mixture has a multimodal molecular weight distribution.

## Revendications

1. Agent cosmétique pour la teinture des fibres kératiniques, contenant au moins deux préparations (A) et (B) conditionnées séparément l'une de l'autre et mélangées immédiatement avant l'utilisation pour former un mélange d'application, dans lequel
- la préparation (A) contient au moins un colorant du groupe constitué des précurseurs de colorants d'oxydation et des colorants directs
- la préparation (B) est une composition fluide contenant de l'eau et au moins un agent oxydant,
**caractérisé en ce que** la préparation (A) est conditionnée dans un film hydrosoluble qui présente, par rapport à son poids total, au moins 60 % en poids d'un mélange polymère comprenant
a1) un copolymère a1) d'alcool vinylique/d'acétate vinylique hydrosoluble et
a2) au moins un polysaccharide hydrosoluble éventuellement modifié ayant une distribution de poids moléculaire multimodale.

2. Agent cosmétique selon la revendication 1, **caractérisé en ce que** le mélange de polymères, par rapport à son poids total, comprend au moins 80 % en poids, de préférence au moins 90 % en poids, en particulier au moins 95 % en poids, d'un mélange comprenant
a1) un copolymère a1) d'alcool vinylique/d'acétate vinylique hydrosoluble et
a2) au moins un polysaccharide hydrosoluble éventuellement modifié, de préférence au moins un polysaccharide hydrosoluble choisi dans le groupe constitué de la méthylcellulose, de la carboxyméthylcellulose, de l'éthylcellulose, de l'hydroxyéthylcellulose, de l'hydroxypropylcellulose, de l'hydroxypropyl-méthylcellulose, de la dextrine et de l'amidon d'hydroxypropyle, notamment au moins un polysaccharide hydrosoluble du groupe constitué de l'amidon d'hydroxypropyle.

3. Agent cosmétique selon l'une quelconque des revendications précédentes, **caractérisée en ce que** le mélange de polymères a un indice de polydispersité supérieur à 2,2, de préférence supérieur à 3,0, en particulier supérieur à 4,6.

4. Agent cosmétique selon l'une quelconque des revendications précédentes, **caractérisée en ce que** le copolymère d'alcool vinylique/d'acétate de vinyle a1) présente un degré d'hydrolyse compris entre 84 % et 90 %, de préférence entre 85 % et 89 % et en particulier entre 86 % et 88 %.

5. Agent cosmétique selon l'une quelconque des revendications précédentes, **caractérisée en ce que** le film hydrosoluble présente une épaisseur de 0,01 à 0,1 mm, de préférence de 0,01 à 0,08 mm et en particulier de 0,02 à 0,06 mm.

6. Agent cosmétique selon l'une quelconque des revendications précédentes, **caractérisée en ce que** le rapport pondéral entre la préparation (A) et la préparation (B) est de 2:1 à 1:3.

7. Agent cosmétique selon l'une quelconque des revendications précédentes, **caractérisée en ce que** la préparation (A), par rapport à son poids, contient de 0,1 à 10 % en poids, de préférence de 0,2 à 8,0 % en poids et en particulier de 0,5 à 6,0 % en poids de colorants du groupe constitué des précurseurs de colorants d'oxydation et des colorants directs, de préférence des précurseurs de colorant d'oxydation.

8. Agent cosmétique selon l'une quelconque des revendications précédentes, **caractérisée en ce que** la préparation (B) contient, par rapport à son poids, moins de 20 % en poids, de préférence moins de 10 % en poids et en particulier moins de 5,0 % de matières grasses.

9. Procédé pour changer la couleur des fibres kératiniques, dans lequel on mélange au moins deux préparations (A) et (B) emballées séparément l'une de l'autre pour former un mélange d'application en introduisant la préparation (A) emballée dans un film hydrosoluble dans la préparation (B) avec dissolution du film hydrosoluble, dans lequel
- la préparation (A) contient au moins un colorant du groupe constitué des précurseurs de colorants d'oxydation et des colorants directs
- la préparation (B) est une composition fluide contenant de l'eau et au moins un agent oxydant,
**caractérisé en ce que** la préparation (A) est conditionnée dans un film hydrosoluble qui présente, par rapport à son poids total, au moins 60 % en poids d'un mélange polymère comprenant
a1) un copolymère a1) d'alcool vinylique/d'acétate vinylique hydrosoluble et
a2) au moins un polysaccharide hydrosoluble éventuellement modifié ayant une distribution de poids moléculaire multimodale.
